(19) Europäisches Patentamt
European Patent Office
Office européen des brevets

(11) Veröffentlichungsnummer : **0 121 490**
**B1**

(12) # EUROPÄISCHE PATENTSCHRIFT

(45) Veröffentlichungstag der Patentschrift : **02.03.88**

(51) Int. Cl.[4] : **C 07 D487/04**, A 61 K 31/50, A 61 K 31/41

(21) Anmeldenummer : **84710009.6**

(22) Anmeldetag : **28.03.84**

(54) **Substituierte 6-Aryl-1,2,4-triazolo[4,3-b]pyridazine, ihre Herstellung und Verwendung.**

(30) Priorität : **31.03.83 DE 3311753**

(43) Veröffentlichungstag der Anmeldung : **10.10.84 Patentblatt 84/41**

(45) Bekanntmachung des Hinweises auf die Patenterteilung : **02.03.88 Patentblatt 88/09**

(84) Benannte Vertragsstaaten : **AT BE CH DE FR GB IT LI LU NL SE**

(56) Entgegenhaltungen :
**EP-A- 0 094 038**
**DE-A- 1 670 095**
**DE-A- 2 113 438**
**DE-A- 2 444 322**
**DE-A- 3 222 342**
**REVUE ROUMAINE DE CHIMIE, Vol. 10, 1965 I. ZUGRAVESCU et al. "Synthesis of pyridazine and benzopyridazine derivatives III" Seiten 641-647**

(73) Patentinhaber : **HOECHST AKTIENGESELLSCHAFT**
**Postfach 80 03 20**
**D-6230 Frankfurt am Main 80 (DE)**

(72) Erfinder : **Rösner, Manfred, Dr.**
**Unter den Buchen 7**
**D-6239 Eppstein/Taunus (DE)**
Erfinder : **Hock, Franz, Dr.**
**Altstadt 19**
**D-6110 Dieburg (DE)**

**EP 0 121 490 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind neue substituierte 6-Aryl-1,2,4-triazolo [4,3-b] pyridazine der Formel I

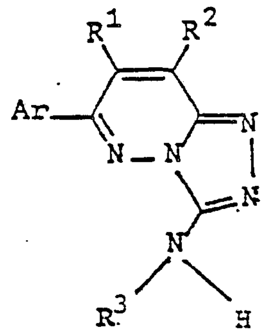

(I)

und deren Salze mit einer physiologisch verträglichen Säure, worin $R^1$ und $R^2$ gleich oder verschieden sind und Wasserstoff, Alkylgruppen mit 1-6 C-Atomen, Phenyl oder Chlor darstellen, worin $R^3$ geradkettiges oder verzweigtes Alkyl mit 1-6 C-Atomen, Cycloalkyl mit 3-8 C-Atomen, Phenylalkyl mit 1-4 C-Atomen im Alkylteil und gegebenenfalls im Phenylteil durch ein, zwei oder drei Fluor, Chlor, Brom, Jod, Trifluormethyl oder Alkyl mit 1-4 C-Atomen substituiert, Alkylcarbonyl mit 1-6 C-Atomen im Alkylteil, Cycloalkylcarbonyl mit 5-7 C-Atomen im Cycloalkylteil gegebenenfalls durch ein, zwei oder drei Fluor, Chlor, Brom, Jod, Trifluormethyl oder Alkyl mit 1-4 C-Atomen substituiertes Benzoyl oder Ar bedeutet, und worin Ar für Phenyl, Phenoxyphenyl, Phenylthiophenyl, 2-Thienyl, 2-Furyl steht, wobei diese Reste gegebenenfalls durch Fluor, Chlor, Alkylgruppen mit 1-6 C-Atomen, Cyano oder Trifluormethyl mono- oder disubstituiert sind, mit Ausnahme derjenigen Verbindung der Formel I, in denen $R^1$ und $R^2$ Wasserstoff, $R^3$ Phenyl und Ar Phenyl oder 4-Methylphenyl bedeuten.

Unter den Verbindungen der Formel I sind solche bevorzugt, in den $R^1$ und $R^2$ gleich oder verschieden sind und Wasserstoff, Methyl, Ethyl, Phenyl oder Chlor, $R^3$ geradkettiges oder verzweigtes Alkyl mit 1-6 C-Atomen, Cycloalkyl mit 3-8 C-Atomen, Phenylalkyl mit 1-4 C-Atomen im Alkylteil und gegebenenfalls im Phenylteil durch ein oder zwei Fluor, Chlor, Brom, Jod, Trifluormethyl oder Alkyl mit 1-4 C-Atomen substituiert oder Ar bedeuten und in denen Ar Phenyl, Phenoxyphenyl, Phenylthiophenyl, 2-Thienyl, 2-Furyl bedeutet, die gegebenenfalls durch ein, zwei oder drei Fluor, Chlor, Trifluormethyl oder Alkylgruppen mit 1-6 C-Atomen substituiert sein können.

Besonders bevorzugt sind solche Verbindungen der Formel I, in denen $R^1$ und $R^2$ gleich oder verschieden sind und Wasserstoff, Methyl oder Ethyl, $R^3$ geradkettiges oder verzweigtes Alkyl mit 1-6 C-Atomen, Cycloalkyl mit 5 oder 6 C-Atomen, Benzyl oder Phenylethyl gegebenenfalls mit ein oder zwei Fluor, Chlor, Trifluormethyl, Methyl oder Ethyl im Phenylring substituiert oder Ar und Ar Phenyl, Phenoxyphenyl, Phenylthiophenyl, 2-Thienyl oder 2-Furyl gegebenenfalls durch ein oder zwei Fluor, Chlor, Trifluormethyl, Methyl oder Ethyl substituiert bedeuten.

Nicht beansprucht werden jedoch diejenigen Verbindungen der Formel I, in denen $R^1$ und $R^2$ Wasserstoff, $R^3$ Phenyl und Ar Phenyl oder 4-Methylphenyl bedeuten.

Diese Verbindungen sind in Rev. Roum. Chim. 10, 641 (1965) und Rev. Med. Chir. 81, 469 (1977) beschrieben worden und sollen teilweise antihypertensiv wirken. Die DE-A-1 670 095 und DE-A-2 113 438 betreffen Triazolo [4,3-b] pyridazine, die in 3-Stellung Nitrofuryl- oder Nitrothienylreste tragen und antimikrobielle Wirksamkeit besitzen. In der DE-A-2 444 322 werden Triazolo [4,3-b]-pyridazine beschrieben, die in 3-Stellung unsubstituiert sind oder in dieser Stellung einen Alkyl- oder substituierten Phenylrest aufweisen, in 6-Stellung tragen sie einen substituierten Aminorest. Für diese Verbindungen wird eine bronchospasmolytische Wirkung angegeben. Die 6-Phenyl-1,2,4-triazolo [4,3-b] pyridazine entsprechend DE-A-2 741 763 tragen in 3-Stellung ein Wasserstoffatom oder eine $C_1$-$C_3$-Alkylgruppe und weisen neben einer anxiolytischen auch eine blutdrucksenkende Wirkung auf. Schließlich werden in der US-Patentschrift 3 708 484 3-Aminotriazolo [4,3-b] pyridazin-Derivate, welche in 7- oder 8-Stellung eine weitere basische Gruppierung tragen, mit antidepressiver und zusätzlich antiinflammatorischer Aktivität beschrieben. Demgegenüber wirken die erfindungsgemäßen Verbindungen der Formel I anxiolytisch und antikonvulsiv.

Gegenstand der Erfindung sind auch Verfahren zur Herstellung dieser Verbindungen sowie pharmazeutische Zubereitungen dieser Verbindungen und ihre Verwendung als Arzneimittel. Das Verfahren zur Herstellung der Verbindungen der Formel I ist dadurch gekennzeichnet, daß man

a) eine Verbindung der Formel II

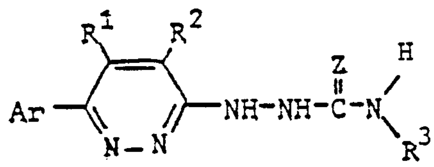

(II)

worin Ar, $R^1$, $R^2$ und $R^3$ die zu Formel I angegebenen Bedeutungen haben und Z für O oder S steht, durch Erhitzen, gegebenenfalls unter Zusatz eines Kondensationsmittels, zu einer Verbindung der Formel I cyclisiert oder

b) eine Verbindung der Formel III, worin $R^6$ Chlor, Brom

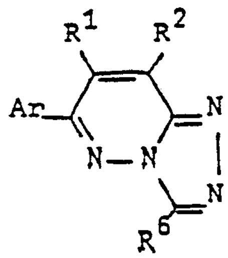

(III)

oder Methylthio bedeutet, und Ar, $R^1$ und $R^2$ die zu Formel I angegebenen Bedeutungen haben, mit einem Amin der Formel IV,

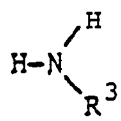

(IV)

worin $R^3$ die zu Formel I gegebene Bedeutung hat, umsetzt, oder

c) eine Verbindung der Formel V oder eines ihrer Salze,

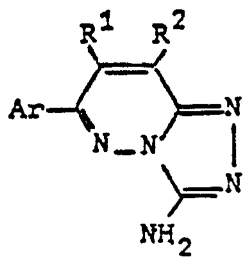

(V)

worin Ar, $R^1$ und $R^2$ die zu Formel I gegebenen Bedeutungen haben, mit einer Verbindung der Formel VI,

$$R^3—Y \quad (VI)$$

worin $R^3$ die zu Formel I gegebene Bedeutung hat und Y für eine Fluchtgruppe wie z. B. Fluor, Chlor, Brom, Jod,

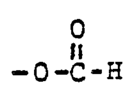

oder den Tosylatrest steht, gegebenenfalls unter Zusatz eines Kondensationsmittels oder Katalysators umsetzt.

Beim Verfahren a) werden die Ausgangsstoffe der Formel II z. B. durch Umsetzung von Arylhydrazinopyridazinen der Formel VII

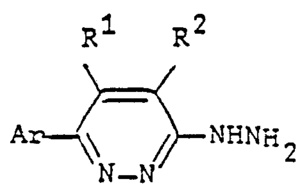

(VII)

worin $R^1$, $R^2$ und Ar die zu Formel I angegebenen Bedeutungen haben, mit Isocyanaten oder Isothiocyanaten der Formel VIII

$$R^3—N=C=Z \qquad (VIII)$$

worin $R^3$ und Z die zu Formel I bzw. II gegebenen Bedeutungen haben, durch Erhitzen auf 40-150 °C, zweckmäßigerweise in einem Lösungsmittel wie z. B. Methanol, Ethanol, Isopropanol, Diisopropylether, Dioxan, Tetrahydrofuran, Toluol, Methylenchlorid, Chloroform oder Dichlorethan, erhalten.

Die Verbindungen der Formel II werden durch Erhitzen, z. B. in einem der genannten Lösungsmittel auf 40-150 °C, gegebenenfalls unter Zusatz eines Kondensationsmittels wie z. B. Eisessig, Cyclohexylcarbodiimid, 1-Hydroxybenztriazol, Phosphoroxychlorid, Phosphoroxychlorid/N,N-Dimethylanilin, Phosphoroxybromid, Phosphorpentachlorid, Thionylchlorid, Quecksilberoxid, Bleioxid in Verbindungen der Formel I übergeführt. Die Verbindungen der Formel II können auch durch Umsetzung von Chlorpyridazinen der Formel IX

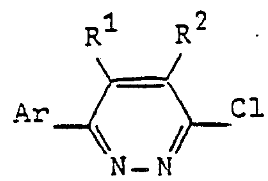

(IX)

worin Ar, $R^1$ und $R^2$ die zu Formel I angegebenen Bedeutungen haben, mit substituierten Semicarbaziden oder Thiosemicarbaziden der Formel X worin $R^3$ und Z die

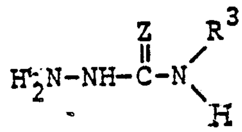

(X)

zur Formel I bzw. II gegebenen Bedeutungen haben, durch Erhitzen, z. B. in einem der genannten Lösungsmittel oder in DMF, DMSO oder Acetonitril auf 40-150 °C erhalten werden. Für das Verfahren b) werden die Ausgangsstoffe der Formel III durch Erhitzen von Arylhydrazinopyridazinen VII mit Ameisensäure oder deren Estern (zu $R^6$ = H), Phosgen, Chlorameisensäureestern, einem Dialkylpyrocarbonat oder einem Dialkylcarbonat (zu $R^6$ = OH) oder mit Schwefelkohlenstoff und Alkali (zu $R^6$ = SH), gegebenenfalls unter Zusatz eines Lösungs- oder Verdünnungsmittels wie Chloroform, Toluol, Dioxan, Essigester, Wasser, Ethanol zu einer Verbindung der Formel III' umgesetzt, in der $R^6$ = H, OH oder SH bedeutet. Durch Erhitzen mit Brom in Eisessig/Natriumacetat (bei $R^6$ = H), Phosphoroxychlorid (bei $R^6$ = OH) bzw. Methyljodid oder Dimethylsulfat (bei R = SH) werden daraus die entsprechenden Verbindungen III mit $R^6$ = Br, Cl, $SCH_3$ erhalten.

Nach Verfahren b) erfolgt die Umsetzung der Verbindungen der Formel III mit Aminen der Formel IV ohne Lösungsmittel oder in einem inerten Lösungsmittel wie Methanol, Isopropanol, 2-Methoxyethanol, Tetrahydrofuran, Dioxan, Toluol, Chloroform, DMF, DMSO, Acetonitril, Aceton oder Essigsäureethylester bei Temperaturen von 20-200 °C, vorzugsweise 50-150 °C bei Normaldruck oder im Autoklaven unter Druck. Die Reaktion mit gasförmigen Aminen kann bei Normaldruck durch Einleiten oder ebenfalls unter Druck durchgeführt werden. Gegebenenfalls kann ein Katalysator wie Kupfer (I)-chlorid oder andere Cu (I)-Salze zur Beschleunigung der Reaktion zugesetzt werden.

Beim Verfahren c) erfolgt die Umsetzung der Verbindungen der Formel V, die z. B. durch Cyclisierung von Arylhydrazinopyridazinen der Formel VII mit Chlorcyan oder Bromcyan erhalten werden, mit Verbindungen der Formel VI ohne oder in Anwesenheit eines Lösungs- oder Verdünnungsmittels wie z. B. Aceton, Methylethylketon, Essigsäureethylester, Toluol, Xylol, Dioxan, Tetrahydrofuran, DMF, DMSO, Acetonitril, Methylenchlorid, Chloroform oder Dichlorethan.

Gegebenenfalls können anorganische oder organische Basen wie z. B. Natriumhydroxid, Triethylamin oder Pyridin zum Binden etwaiger bei der Reaktion entstehender Säuren oder auch, insbesondere bei der Reaktion mit Säureanhydriden, katalytische Mengen einer starken Säure wie z. B. Salzsäure, Schwefelsäure oder Trifluoressigsäure zugesetzt werden.

Arylhydrazinopyridazine der Formel VII sind z. B. aus J. Heterocyclic Chem. 15, 881 (1978) bekannt oder können aus Chlorverbindungen der Formel IX mit Hydrazinhydrat nach literaturbekannten Verfahren (The Chemistry of Heterocyclic Compounds, Vol. 28 Pyridazines, Editors A. Weissberger und E.C. Taylor, John Wiley, New York 1973) hergestellt werden. Beide Literaturstellen beschreiben auch die Synthese der Chlorverbindungen IX und ihrer Vorstufen.

Wenn die Verbindungen der Formel I nach den beschriebenen Verfahren als Salze erhalten werden,

so kann daraus mit Ammoniak, Aminen oder Hydroxiden die zugehörige Base freigesetzt werden. Die freien Basen der Formel I können mit physiologisch verträglichen Säuren in die entsprechenden Salze übergeführt werden. Als Säuren kommen anorganische oder organische Säuren in Betracht wie Chlor- oder Bromwasserstoffsäure, Phosphorsäure, Essigsäure, Benzoesäure, Citronensäure, Maleinsäure, Fumarsäure, Milchsäure, Weinsäure, Bernsteinsäure, Acetylglycin.

Die erfindungsgemäßen Verbindungen der Formel I sind zur Herstellung von Arzneimitteln geeignet. Die Arzneimittel können eine oder mehrere der erfindungsgemäßen Verbindungen oder auch Mischungen derselben mit anderen pharmazeutisch wirksamen Stoffen enthalten. Zur Herstellung der Arzneimittel können die üblichen pharmazeutischen Träger- und Hilfsstoffe und bekannte galenische Verfahren verwendet werden. Die Arzneimittel können enteral, parenteral, oral oder perlingual angewendet werden. Beispielsweise kann die Verabreichung in Form von Tabletten, Kapseln, Pillen, Dragees, Zäpfchen, Gelees, Cremes, Puder, Liquida, Stäubepulver oder Aerosolen erfolgen. Als Liquida kommen zum Beispiel in Frage : ölige oder wäßrige Lösungen oder Suspensionen, Emulsionen, injizierbare Lösungen oder Suspensionen.

Die erfindungsgemäßen Verbindungen können außerdem als Zwischenprodukte zur Herstellung anderer Arzneimittel Verwendung finden.

Als erfindungsgemäße Verbindungen seien genannt : (Für den Stammnamen « 1,2,4-triazolo [4,3-b] pyridazin » wird im folgenden die Abkürzung « TP » verwendet.)

6-(3-Fluorphenyl)-3-phenylamino-TP
6-(4-Fluorphenyl)-3-phenylamino-TP
6-(3-Trifluormethylphenyl)-3-phenylamino-TP
6-(3,4-Difluorphenyl)-3-phenylamino-TP
6-(4-Phenoxyphenyl)-3-phenylamino-TP
6-(4-Phenylthiophenyl)-3-phenylamino-TP
6-(4-(4-Fluorphenoxy)-phenyl)-3-phenylamino-TP
6-(3-Chlorphenyl)-3-phenylamino-TP
6-(5-Chlor-2-thienyl)-3-phenylamino-TP
6-(3-Fluorphenyl)-3-(subst. phenylamino)-TP
6-(4-Fluorphenyl)-3-(subst. phenylamino)-TP
6-(3-Trifluormethylphenyl)-3-(subst. phenylamino)-TP
6-(3,4-Difluorphenyl)-3-(subst. phenylamino)-TP
6-(4-Phenoxyphenyl)-3-(subst. phenylamino)-TP
6-(4-Phenylthiophenyl)-3-(subst. phenylamino)-TP
6-(4-(4-Fluorphenoxy)-phenyl)-3-(subst. phenylamino)-TP
6-(3-Chlorphenyl)-3-(subst. phenylamino)-TP
6-(5-Chlor-2-thienyl)-3-(subst. phenylamino)-TP

worin « subst. phenyl » insbesondere für 2-, 3- oder 4-fluorphenyl, 2-, 3- oder 4-chlorphenyl, 2-, 3- oder 4-trifluormethylphenyl, 3,4-dichlor- oder 3,4-difluorphenyl, 2,4-dichlor- oder 2,4-difluorphenyl, 2,5-dichlor- oder 2,5-difluorphenyl, 3,5-dichlor- oder 3,5-difluorphenyl, 2,6-dichlor- oder 2,6-difluorphenyl steht.

6-(3-Fluorphenyl)-3-acetamino-TP
6-(4-Fluorphenyl)-3-acetamino-TP
6-(3-Trifluormethylphenyl)-3-acetamino-TP
6-(3,4-Difluorphenyl)-3-acetamino-TP
6-(4-Phenoxyphenyl)-3-acetamino-TP
6-(4-Phenylthiophenyl)-3-acetamino-TP
6-(4-(4-Fluorphenoxy)-phenyl)-3-acetamino-TP
6-(3-Chlorphenyl)-3-acetamino-TP
6-(5-Chlor-2-thienyl)-3-acetamino-TP
6-(3-Fluorphenyl)-3-benzoylamino-TP
6-(4-Fluorphenyl)-3-benzoylamino-TP
6-(3-Trifluormethylphenyl)-3-benzoylamino-TP
6-(3,4-Difluorphenyl)-3-benzoylamino-TP
6-(4-Phenoxyphenyl)-3-benzoylamino-TP
6-(4-Phenylthiophenyl)-3-benzoylamino-TP
6-(4-(4-Fluorphenoxy)-phenyl)-3-benzoylamino-TP
6-(3-Chlorphenyl)-3-benzoylamino-TP
6-(5-Chlor-2-thienyl)-3-benzoylamino-TP
6-(3-Fluorphenyl)-3-(subst. benzoylamino)-TP
6-(4-Fluorphenyl)-3-(subst. benzoylamino)-TP
6-(3-Trifluormethylphenyl)-3-(subst. benzoylamino)-TP
6-(3,4-Difluorphenyl)-3-(subst. benzoylamino)-TP
6-(4-Phenoxyphenyl)-3-(subst. benzoylamino)-TP
6-(4-Phenylthiophenyl)-3-(subst. benzoylamino)-TP
6-(4-(4-Fluorphenoxy)-phenyl)-3-(subst. benzoylamino)-TP
6-(3-Chlorphenyl)-3-(subst. benzoylamino)-TP

6-(5-Chlor-2-thienyl)-3-(subst. benzoylamino)-TP
worin « subst. benzoyl » insbesondere für 2-, 3- oder 4-fluorbenzoyl, 2-, 3- oder 4-chlorbenzoyl, 2-, 3- oder 4-brombenzoyl, 2-, 3- oder 4-trifluormethylbenzoyl, 3,4-dichlor- oder 3,4-difluorbenzoyl, 2,5-dichlor- oder 2,5-difluorbenzoyl, 3,5-dichlor- oder 3,5-difluorbenzoyl, 2,6-dichlor- oder 2,6-difluorbenzoyl steht.

Die erfindungsgemäßen Verbindungen der Formel I wirken auf das Zentralervensystem ; insbesondere wirken sie anxiolytisch und antikonvulsiv. Als Indikationen kommen deshalb Schlaflosigkeit, Erregung und vegetative Verstimmung in Betracht.

Die pharmazeutischen Zubereitungen enthalten im allgemeinen zwischen 1 bis 10 % der erfindungsgemäßen aktiven Komponente(n).

Die anxiolytische Wirkung der Verbindungen der Formel I ist von einer sehr geringen Sedierung und guten Verträglichkeiten begleitet ($LD_{50} > 300$ mg/kg i. p. an der Maus). Dies geht aus Untersuchungen hervor, bei denen der Einfluß der erfindungsgemäßen Verbindungen auf die motorische Aktivität, die Hexobarbital-Narkose und den Cardiazolkrampf bei Mäusen gemessen wurde. Außerdem wurde noch der Geller-Anxiolysetest sowie der Lick-Shock-Test an Ratten herangezogen.

Die niedrigste bereits wirksame Dosis in den oben angegebenen Versuchen ist beispielsweise 5 mg/kg oral, 2,5 mg/kg sublingual, 1 mg/kg intravenös. Als allgemeiner Dosisbereich für Wirkung (Tierversuch wie oben) kommt beispielsweise in Frage : 5 bis 50 mg/kg oral, 2,5 bis 25 mg/kg sublingual, 1 bis 10 mg/kg intravenös.

Beispielsweise können 3 mal täglich 1 bis 3 Tabletten mit einem Gehalt von 10 bis 100 mg wirksamer Substanz oder zum Beispiel bei intravenöser Injektion 1 bis 3 mal täglich eine Ampulle von 2 bis 4 ml Inhalt mit 0,5 bis 5 mg Substanz appliziert werden.

## Beispiel 1

3-Cyclohexylamino-6-phenyl-1,2,4-triazolo [4,3-b] pyridazin-hydrochlorid

5 g 3-Hydrazino-6-phenylpyridazin (Formel VII, Fp. 146 °C) werden in 50 ml Ethanol heiß gelöst und mit 3,8 g Cyclohexylisothiocyanat versetzt. Man rührt zwei Stunden unter Rückfluß, kühlt auf 0 °C, saugt ab, wäscht mit Ethanol und trocknet. Das entstandene Zwischenprodukt (Formel II, Z = S) wird mit 6,1 g Dicyclohexylcarbodiimid in 50 ml 2-Methoxyethanol 5 Stunden zum Rückfluß erhitzt. Die Reaktionslösung wird unter vermindertem Druck zur Trockne eingedampft und zweimal aus Isopropanol kristallisiert. Durch Versetzen mit ethanolischer Salzsäure erhält man das Hydrochlorid, das abgesaugt, mit Ethanol gewaschen und getrocknet wird, Fp. 268 °C.

## Beispiel 2

3-Benzylamino-6-phenyl-1,2,4-triazolo [4,3-b] pyridazinhydrochlorid

8 g 3-Hydrazino-6-phenylpyridazin werden in 70 ml Ethanol heiß gelöst, mit 7 g Benzylisothiocyanat versetzt und eine Stunde unter Rückfluß gerührt. Die Zwischenstufe (Formel II Z = S) wird nach dem Abkühlen abgesaugt, mit Ethanol gewaschen, getrocknet und anschließend mit 8,9 g Dicyclohexylcarbodiimid in 50 ml 2-Methoxyethanol 5 Stunden zum Rückfluß erhitzt.

Nach dem Einengen wird mit ethanolischer Salzsäure versetzt, eingedampft und einmal aus Eisessig und einmal aus Toluol umkristallisiert und mit Diisopropylether gewaschen, Fp. 236 °C (Zers.).

## Beispiel 3

6-(4-Fluorphenyl)-3-methylamino-1,2,4-triazolo [4,3-b] pyridazin-hydrochlorid

Zu 5 g 3-(4-Fluorphenyl)-6-hydrazinopyridazin (Formel VII, Fp. 194 °C) in 40 ml siedendem Dioxan werden 1,65 ml Methylisocyanat in 10 ml Dioxan getropft. Man erhitzt noch zwei Stunden zum Rückfluß, engt die Lösung unter vermindertem Druck ein, verrührt mit Ethanol, saugt ab und trocknet. Das Zwischenprodukt (Formel II, Z = O) wird mit Phosphoroxychlorid 4 Stunden zum Rückfluß erhitzt. Die Reaktionslösung wird vorsichtig mit Eis hydrolysiert. Das ausfallende Hydrochlorid des Produkts wird abgesaugt, mit Wasser gewaschen, mit Isopropanol ausgekocht, abgesaugt und getrocknet, Fp. 297 °C.

## Beispiel 4

6-(4-Fluorphenyl)-3-phenylamino-1,2,4-triazolo [4,3-b] pyridazin

5 g 3-(4-Fluorphenyl)-6-hydrazinopyridazin und 3,7 g Phenylisothiocyanat werden in 50 ml Ethanol 3 Stunden unter Rückfluß gerührt. Nach Abkühlen wird das Zwischenprodukt (Formel II, Z = S) abgesaugt, mit Ethanol gewaschen und getrocknet, Fp. 223 °C (Sintern).

7,5 g dieses Zwischenprodukts werden mit 5 g Dicyclohexylcarbodiimid in 50 ml 2-Methoxyethanol 2 Stunden zum Rückfluß erhitzt. Die beim Abkühlen entstehende Fällung wird abgesaugt, mit Ethanol

gewaschen und aus Isopropanol umkristallisiert, Fp. 265 °C.

Beispiel 5

3-(4-Chlorphenylamino)-6-(4-fluorphenyl)-1,2,4-triazolo [4,3-b] pyridazin

Zu 5 g 3-(4-Fluorphenyl)-6-hydrazinopyridazin in 40 ml Dioxan tropft man 4,1 g 4-Chlorphenylisocyanat gelöst in 15 ml Dioxan und erhitzt 2,5 Stunden zum Rückfluß. Nach dem Abkühlen saugt man ab, wäscht mit Isopropanol und trocknet, Fp. 223 °C.

5 g dieses Zwischenprodukts (Formel II, Z = O) werden in 40 ml trockenem Toluol mit 3,5 ml N,N-Dimethylanilin und 1,4 ml Phosphoroxychlorid 3 Stunden zum Rückfluß erhitzt. Nach dem Abkühlen wird das Toluol abdekantiert und der verbleibende, ölige Rückstand mit 2n-HCl verrührt. Man saugt den Feststoff (Hydrochlorid, Fp. 286 °C) ab, kocht ihn mit halbkonzentriertem Ammoniak auf und saugt nach dem Abkühlen die Base ab und kristallisiert aus Isopropanol um, Fp. 257 °C.

Beispiel 6

3-(3-Fluorphenylamino)-6-(3-trifluormethylphenyl)-1,2,4-triazolo [4,3-b] pyridazin

5 g 3-Hydrazino-6-(3-trifluormethylphenyl)-pyridazin (Formel VII, Fp. 167 °C) werden in 40 ml Ethanol auf etwa 50 °C erwärmt. Man tropft 3,3 g 3-Fluorphenylisothiocyanat, gelöst in 10 ml Ethanol, zu, erhitzt 3 Stunden zum Rückfluß und saugt nach dem Abkühlen den entstandenen Niederschlag ab. Nach Waschen mit Ethanol und Trocknen werden 6 g des Zwischenprodukts (Formel II, Z = S), Fp. 164 °C, in 40 ml Toluol mit 3,8 ml N,N-Dimethylanilin und 1,5 ml Phosphoroxychlorid 3 Stunden zum Rückfluß erhitzt. Nach dem Abkühlen wird die Toluolphase abdekantiert und der verbleibende, ölige Rückstand mit 2n-HCl verrührt. Der dabei entstehende Feststoff (Hydrochlorid) wird abgesaugt, mit halbkonzentriertem Ammoniak aufgekocht und kalt abgesaugt (Base). Nach Umkristallisation aus Eisessig/Wasser, Fp. 209 °C.

Beispiel 7

3-(4-Fluorphenylamino)-6-(4-phenylthio-phenyl)-1,2,4-triazolo [4,3-b] pyridazin-hydrochlorid

5 g 3-Hydrazino-6-(4-phenylthio-phenyl)-pyridazin (Formel VII, Fp. 142 °C) werden in 40 ml Ethanol zum Rückfluß erhitzt. Man tropft 2,9 g 4-Fluorphenylisothiocyanat in 10 ml Ethanol langsam zu und erhitzt insgesamt 3 Stunden zum Rückfluß. Nach dem Abkühlen wird die Fällung abgesaugt, mit Ethanol gewaschen und getrocknet, Fp. 188 °C. 6 g dieses Zwischenprodukts (Formel II, Z = S) werden mit 3 g Dicyclohexylcarbodiimid in 40 ml 2-Methoxyethanol 5 Stunden lang unter Rückfluß gerührt. Nach dem Abkühlen saugt man ab und wäscht mit Isopropanol. Der Niederschlag wird mit ethanolischer Salzsäure aufgekocht, eingeengt, abgesaugt, mit Ethanol gewaschen und getrocknet, Fp. 263 °C.

Analog zu den Beispielen 1-7 können die folgenden Verbindungen hergestellt werden :

Der Stammname «1,2,4-Triazolo [4,3-b] pyridazin» wird im folgenden als «TP» abgekürzt.

8. 3-Methylamino-6-phenyl-TP-HCl Fp. 254 °C.
9. 3-Propylamino-6-phenyl-TP-HCl Fp. 206 °C.
10. 3-Hexylamino-6-phenyl-TP-HCl Fp. 164 °C.
11. 3-(3-Fluorphenylamino)-6-phenyl-TP-HCl Fp. 266 °C (Zers.).
12. 3-(4-Fluorphenylamino)-6-phenyl-TP-HCl Fp. 264 °C.
13. 3-(3-Chlorphenylamino)-6-phenyl-TP-HCl Fp. 260 °C (Zers.).
14. 3-(4-Chlorphenylamino)-6-phenyl-TP-HCl Fp. 258 °C (Zers.).
15. 3-(3-Trifluormethylphenylamino)-6-phenyl-TP-HCl Fp. 245 °C.
16. 3-Propylamino-6-(4-fluorphenyl)-TP-HCl Fp. 241 °C.
17. 3-Cyclohexylamino-6-(4-fluorphenyl)-TP-HCl 262 °C (Zers.).
18. 3-(3-Chlorphenylamino)-6-(4-fluorphenyl)-TP-HCl Fp. 250 °C.
19. 3-(3,4-Dichlorphenylamino)-6-(4-fluorphenyl)-TP-HCl Fp. 289-290 °C.
20. 3-(2-Fluorphenylamino)-6-(4-fluorphenyl)-TP-HCl Fp. 242 °C.
21. 3-(3-Fluorphenylamino)-6-(4-fluorphenyl)-TP-HCl Fp. 260-262 °C.
22. 3-(4-Fluorphenylamino)-6-(4-fluorphenyl)-TP-HCl Fp. 282-284 °C.
23. 3-(4-Methylphenylamino)-6-(4-fluorphenyl)-TP-HCl Fp. 263 °C.
24. 3-(3-Cyanophenylamino)-6-(4-fluorphenyl)-TP-HCl Fp. 312 °C.
25. 3-(3-Trifluormethylphenylamino)-6-(4-fluorphenyl)-TP-HCl Fp. 286 °C.
26. 3-Phenylamino-6-(3-fluorphenyl)-TP-HCl Fp. 266° (Zers.).
27. 3-(4-Chlorphenylamino)-6-(3-fluorphenyl)-TP Fp. 258 °C.
28. 3-(3-Fluorphenylamino)-6-(3-fluorphenyl)-TP Fp. 236 °C.
29. 3-(4-Fluorphenylamino)-6-(3-fluorphenyl)-TP Fp. 241 °C.
30. 3-Phenylamino-6-(3-trifluormethylphenyl)-TP Fp. 206 °C.
31. 3-(3-Chlorphenylamino)-6-(3-trifluormethylphenyl)-TP Fp. 212 °C.

32. 3-(4-Chlorphenylamino)-6-(3-trifluormethylphenyl)-TP Fp. 208 °C. .
33. 3-(3,4-Dichlorphenylamino)-6-(3-trifluormethylphenyl)-TP Fp. 198 °C.
34. 3-(2-Fluorphenylamino)-6-(3-trifluormethylphenyl)-TP Fp. 198 °C.
35. 3-(3-Fluorphenylamino)-6-(3-trifluormethylphenyl)-TP Fp. 209 °C.
36. 3-(4-Fluorphenylamino)-6-(3-trifluormethylphenyl)-TP Fp. 234 °C.
37. 3-Phenylamino-6-(4-trifluormethylphenyl)-TP-HCl Fp. 271 °C.
38. 3-(3-Fluorphenylamino)-6-(4-phenoxyphenyl)-TP Fp. 211 °C.
39. 3-Phenylamino-6-(4-phenylthio-phenyl)-TP-HCl Fp. 247 °C.
40. 3-(4-Chlorphenylamino)-6-(4-phenylthio-phenyl)-TP-HCl Fp. 274 °C.
41. 3-(3-Fluorphenylamino)-6-(4-phenylthio-phenyl)-TP-HCl Fp. 253 °C.
42. 3-(4-Fluorphenylamino)-6-(4-phenylthio-phenyl)-TP-HCl Fp. 263 °C.
43. 3-Phenylamino-6-(5-chlor-2-thienyl)-TP-HCl Fp. 271 °C.
44. 3-(4-Chlorphenylamino)-6-(5-chlor-2-thienyl)-TP Fp. 246 °C.
45. 3-(3-Fluorphenylamino)-6-(5-chlor-2-thienyl)-TP Fp. 287 °C.
46. 3-(2-Fluorphenylamino)-6-(5-chlor-2-thienyl)-TP Fp. 232 °C.
47. 6-[4-(4-Fluorphenoxy)-phenyl]-3-phenylamino-TP Fp. 235 °C.
48. 6-[4-(4-Fluorphenoxy)-phenyl]-3-(3-fluorphenylamino)-TP Fp. 233 °C.
49. 3-(4-Chlorphenylamino)-6-[4-(4-fluorphenoxy)-phenyl]-TP Fp. 267 °C.
50. 3-(3-Fluorphenylamino)-6-(2-furyl)-TP Fp. 217-218 °C.
51. 3-(4-Chlorphenylamino)-6-(2-furyl)-TP Fp. 201-202 °C.
52. 3-Phenylamino-6-(2-thienyl)-TP Fp. 204 °C.
53. 3-(3-Fluorphenylamino-6-(2-thienyl)-TP Fp. 212 °C.

Beispiel 54 Verfahren b)

3-Cyclohexylamino-6-phenyl-1,2,4-triazolo [4,3-b] pyridazinhydrochlorid

3 g 3-Brom-6-phenyl-1,2,4-triazolo [4,3-b] pyridazin werden in 10 ml Cyclohexylamin fünf Stunden lang zum Rückfluß erhitzt. Nach dem Abkühlen verrührt man mit Diisopropylether, saugt ab und kristallisiert aus Isopropanol um. Mit ethanolischer Salzsäure erhält man das Hydrochlorid, Fp. 258 °C.

In analoger Verfahrensweise erhält man die erfindungsgemäßen Verbindungen der Beispiele 2 bis 53.

Dabei wird ein in Analogie zu Literaturverfahren hergestelltes 6-Aryl-3-brom-1,2,4-triazolo [4,3-b] pyridazin (allg. Formel III), das den gleichen 6-Substituenten trägt wie die Verbindung des betreffenden Beispiels, mit demjenigen Amin (Formel IV) umgesetzt, das dem 3-Substituenten des Beispiels zugrundeliegt.

Beispiel 55 Verfahren c)

3-Benzylamino-6-phenyl-1,2,4-triazolo [4,3-b] pyridazin-hydrochlorid

5 g 3-Amino-6-phenyl-1,2,4-triazolo [4,3-b] pyridazin und 3 g Benzylchlorid werden in 25 ml Dimethylformamid mit 5 g Kaliumcarbonat 10 Stunden am Rückfluß erhitzt. Nach dem Abkühlen wird mit Wasser versetzt, abgesaugt und mit ethanolischer Salzsäure versetzt und eingedampft. Nach Umkristallisation aus Eisessig wird mit Diisopropylether gewaschen und getrocknet, Fp. 236 °C (Zers.).

In analoger Verfahrensweise erhält man die erfindungsgemäßen Verbindungen der Beispiele 1 und 3 bis 53.

Dabei wird das dem jeweiligen Beispiel zugrundeliegende 3-Amino-6-aryl-1,2,4-triazolo [4,3-b] pyridazin (Formel V, beschrieben in der DE-OS 32 17 325) mit demjenigen $R^3$—Y umgesetzt (Formel VI), dessen Alkyl- oder Arylrest dem 3-Substituenten des betreffenden Beispiels zugrundeliegt.

**Patentansprüche** (für die Vertragsstaaten : BE, CH, DE, FR, GB, IT, LI, LU, NL, SE)

1. Verbindungen der Formel I

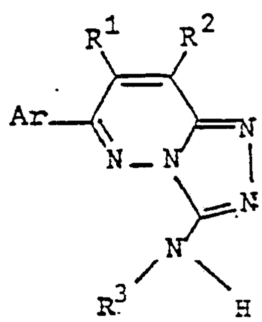

(I)

und deren Salze mit einer physiologisch verträglichen Säure, worin $R^1$ und $R^2$ gleich oder verschieden sind und Wasserstoff, Alkylgruppen mit 1-6 C-Atomen, Phenyl oder Chlor darstellen, worin $R^3$ geradkettiges oder verzweigtes Alkyl mit 1-6 C-Atomen, Cycloalkyl mit 3-8 C-Atomen, Phenylalkyl mit 1-4 C-Atomen im Alkylteil und gegebenenfalls im Phenylteil durch ein, zwei oder drei Fluor, Chlor, Brom, Jod, Trifluormethyl oder Alkyl mit 1-4 C-Atomen substituiert, Alkylcarbonyl mit 1-6 C-Atomen im Alkylteil, Cycloalkylcarbonyl mit 5-7 C-Atomen im Cycloalkylteil, gegebenenfalls durch ein, zwei oder drei Fluor, Chlor, Brom, Jod, Trifluormethyl oder Alkyl mit 1-4 C-Atomen substituiertes Benzoyl oder Ar bedeutet, und worin Ar für Phenyl, Phenoxyphenyl, Phenylthiophenyl, 2-Thienyl oder 2-Furyl steht, wobei diese Reste gegebenenfalls durch Fluor, Chlor, Alkylgruppen mit 1-6 C-Atomen, Cyano oder Trifluormethyl mono- oder disubstituiert sind, mit Ausnahme derjenigen Verbindungen der Formel I, in denen $R^1$ und $R^2$ Wasserstoff, $R^3$ Phenyl und Ar Phenyl oder 4-Methylphenyl bedeuten.

2. Verfahren zur Herstellung von Verbindungen der Formel I in Anspruch 1, dadurch gekennzeichnet, daß man

a) eine Verbindung der Formel II

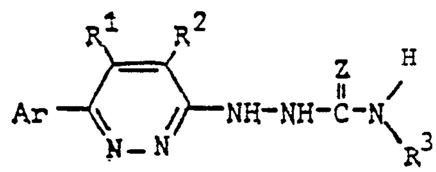

(II)

worin Ar, $R^1$, $R^2$ und $R^3$ die zu Formel I angegebenen Bedeutungen haben und Z für O oder S steht, durch Erhitzen, gegebenenfalls unter Zusatz eines Kondensationsmittels, zu einer Verbindung der Formel I cyclisiert, oder

b) eine Verbindung der Formel III, worin $R^6$ Chlor, Brom

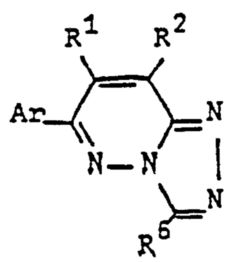

(III)

oder Methylthio bedeutet, und Ar, $R^1$ und $R^2$ die zu Formel I angegebenen Bedeutungen haben, mit einem Amin der Formel IV,

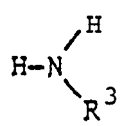

(IV)

worin $R^3$ die zu Formel I gegebene Bedeutung hat, umsetzt, oder

c) eine Verbindung der Formel V oder eines ihrer Salze,

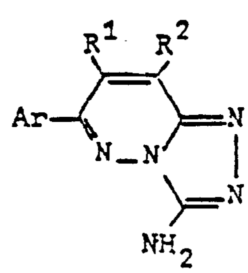

(V)

worin Ar, $R^1$ und $R^2$ die zu Formel I gegebenen Bedeutungen haben, mit einer Verbindung der Formel VI,

$R^3—Y$ (VI)

worin $R^3$ die zu Formel I gegebene Bedeutung hat und Y für eine Fluchtgruppe wie z. B. Fluor, Chlor, Brom, Jod,

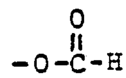

oder den Tosylatrest steht, gegebenenfalls unter Zusatz eines Kondensationsmittels oder Katalysators umsetzt, und gegebenenfalls eine so erhaltene Verbindung der Formel I durch Zusetzen einer physiologisch verträglichen Säure in das Salz überführt.

3. Pharmazeutische Präparate mit anxiolytischer und antikonvulsiver Wirkung, gekennzeichnet durch einen Gehalt an einer Verbindung der Formel I in Anspruch 1.

4. Verbindungen der Formel I gemäß Anspruch 1 zur Behandlung von Erregungszuständen, Schlaflosigkeit, Krampfzuständen und vegetativer Dystonie.

**Patentanspruch** (für den Vertragsstaat AT)

Verfahren zur Herstellung von Verbindungen der Formel I

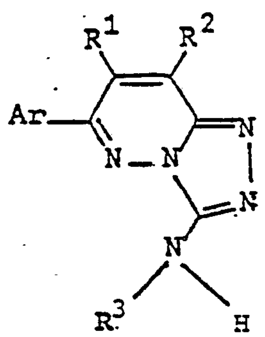

(I)

und deren Salze mit einer physiologisch verträglichen Säure, worin $R^1$ und $R^2$ gleich oder verschieden sind und Wasserstoff, Alkylgruppen mit 1-6 C-Atomen, Phenyl oder Chlor darstellen, worin $R^3$ geradkettiges oder verzweigtes Alkyl mit 1-6 C-Atomen, Cycloalkyl mit 3-8 C-Atomen, Phenylalkyl mit 1-4 C-Atomen im Alkylteil und gegebenenfalls im Phenylteil durch ein, zwei oder drei Fluor, Chlor, Brom, Jod, Trifluormethyl oder Alkyl mit 1-4 C-Atomen substituiert, Alkylcarbonyl mit 1-6 C-Atomen im Alkylteil, Cycloalkylcarbonyl mit 5-7 C-Atomen im Cycloalkylteil, gegebenenfalls durch ein, zwei oder drei Fluor, Chlor, Brom, Jod, Trifluormethyl oder Alkyl mit 1-4 C-Atomen substituiertes Benzoyl oder Ar bedeutet, und worin Ar für Phenyl, Phenoxyphenyl, Phenylthiophenyl, 2-Thienyl oder 2-Furyl steht, wobei die Reste gegebenenfalls durch Fluor, Chlor, Alkylgruppen mit 1-6 C-Atomen, Cyano oder Trifluormethyl mono- oder disubstituiert sind, mit Ausnahme der Verbindungen der Formel I, in denen $R^1$ und $R^2$ Wasserstoff, $R^3$ Phenyl und Ar Phenyl oder 4-Methylphenyl bedeuten, dadurch gekennzeichnet, daß man

a) eine Verbindung der Formel II

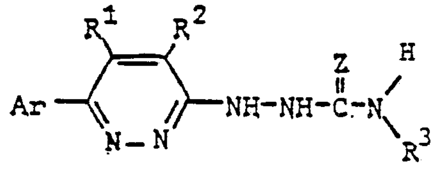

(II)

worin Ar, $R^1$, $R^2$ und $R^3$ die zu Formel I angegebenen Bedeutungen haben und Z für O oder S steht, durch Erhitzen, gegebenenfalls unter Zusatz eines Kondensationsmittels, zu einer Verbindung der Formel I cyclisiert, oder

b) eine Verbindung der Formel III, worin $R^6$ Chlor, Brom

(Siehe Formel Seite 11 f.)

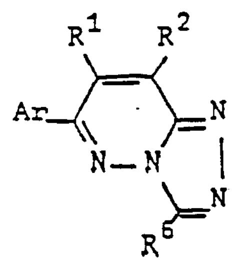

(III)

oder Methylthio bedeutet, und Ar, $R^1$ und $R^2$ die zu Formel I angegebenen Bedeutungen haben, mit einem Amin der Formel IV

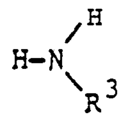

(IV)

worin $R^3$ die zu Formel I gegebene Bedeutung hat, umsetzt, oder

c) eine Verbindung der Formel V oder eines ihrer Salze,

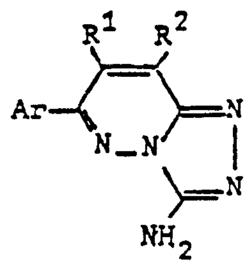

(V)

worin Ar, $R^1$ und $R^2$ die zu Formel I gegebenen Bedeutungen haben, mit einer Verbindung der Formel VI

$$R^3—Y \qquad (VI)$$

worin $R^3$ die zu Formel I gegebene Bedeutung hat und Y für eine Fluchtgruppe wie z. B. Fluor, Chlor, Brom, Jod,

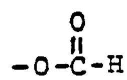

oder den Tosylatrest steht, gegebenenfalls unter Zusatz eines Kondensationsmittels oder Katalysators umsetzt, und gegebenenfalls eine so erhaltene Verbindung der Formel I durch Zusetzen einer physiologisch verträglichen Säure in das Salz überführt.

**Claims** (for the Contracting States : BE, CH, DE, FR, GB, IT, LI, LU, NL, SE)

1. Compounds of the formula

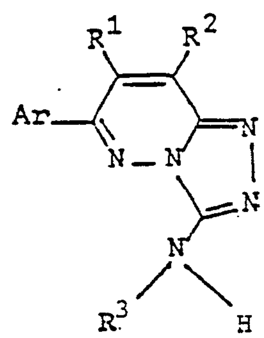

(I)

and salts thereof with a physiologically acceptable acid, in which formula $R^1$ and $R^2$ are identical or different and represent hydrogen, alkyl groups having 1-6 carbon atoms, phenyl or chlorine, $R^3$ denotes linear or branched alkyl having 1-6 carbon atoms, cycloalkyl having 3-8 carbon atoms, phenylalkyl which

has 1-4 carbon atoms in the alkyl part and is optionally monosubstituted, disubstituted or trisubstituted in the phenyl part by fluorine, chlorine, bromine, iodine, trifluoromethyl or alkyl having 1-4 carbon atoms, alkylcarbonyl having 1-6 carbon atoms in the alkyl part, cycloalkylcarbonyl having 5-7 carbon atoms in the cycloalkyl part, or benzoyl which is optionally monosubstituted, disubstituted or trisubstituted by fluorine, chlorine, bromine, iodine, trifluoromethyl or alkyl having 1-4 carbon atoms, or denotes Ar, and in which Ar represents phenyl, phenoxyphenyl, phenylthiophenyl, 2-thienyl or 2-furyl, these radicals being optionally monosubstituted or disubstituted by fluorine, chlorine, alkyl groups having 1-6 carbon atoms, cyano or trifluoromethyl, with the exception of compounds of the formula I in which $R^1$ and $R^2$ denote hydrogen, $R^3$ denotes phenyl and Ar denotes phenyl or 4-methylphenyl.

2. A process for the preparation of a compound of the formula I as claimed in Claim 1, which comprises

a) cyclizing a compound of the formula II

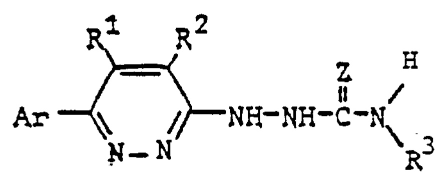

(II)

in which Ar, $R^1$, $R^2$ and $R^3$ have the meanings indicated for formula I and Z represents O or S, by heating, if appropriate with the addition of a condensation agent, to give a compound of the formula I, or

b) by reacting a compound of the formula III

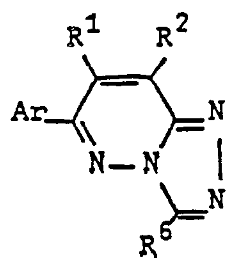

(III)

in which $R^6$ denotes chlorine, bromine or methylthio and Ar, $R^1$ and $R^2$ have the meanings indicated for formula I, with an amine of the formula IV

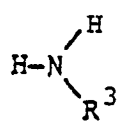

(IV)

in which $R^3$ has the meaning indicated for formula I, or

c) reacting, if appropriate with the addition of a condensation agent or catalyst, a compound of the formula V

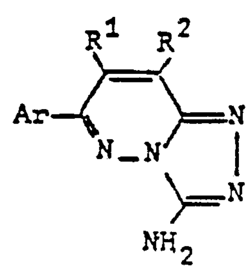

(V)

or one of its salts in which Ar, $R^1$ and $R^2$ have the meanings indicated for formula I, with a compound of the formula VI

$$R^3\text{—}Y \qquad (VI)$$

in which $R^3$ has the meaning indicated for formula I and Y represents a leaving group, such as, for example, fluorine, chlorine, bromine, iodine,

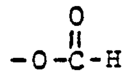

or the tosylate radical, and, if appropriate, converting a compound thus obtained into a salt thereof by adding a physiologically acceptable acid.

3. A pharmaceutical formulation having an anxiolytic and anticonvulsive action, which contains a compound of the formula I as claimed in Claim 1.

4. A compound of the formula I as claimed in Claim 1 for the treatment of states of nervous tension, sleeplessness, states of spasm and neuro-dystonia.

**Claim** (for the Contracting State AT)

A process for the preparation of compounds of the formula I

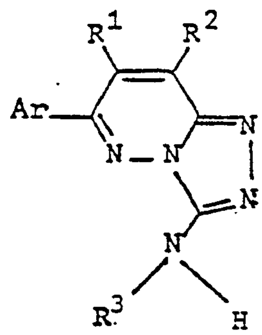

(I)

and salts thereof with a physiologically acceptable acid, in which formula $R^1$ and $R^2$ are identical or different and represent hydrogen, alkyl groups having 1-6 carbon atoms, phenyl or chlorine, $R^3$ denotes linear or branched alkyl having 1-6 carbon atoms, cycloalkyl having 3-8 carbon atoms, phenylalkyl which has 1-4 carbon atoms in the alkyl part and is optionally monosubstituted, disubstituted or trisubstituted in the phenyl part by fluorine, chlorine, bromine, iodine, trifluoromethyl or alkyl having 1-4 carbon atoms, alkylcarbonyl having 1-6 carbon atoms in the alkyl part, cycloalkylcarbonyl having 5-7 carbon atoms in the cycloalkyl part, or benzoyl which is optionally monosubstituted, disubstituted or trisubstituted by fluorine, chlorine, bromine, iodine, trifluoromethyl or alkyl having 1-4 carbon atoms, or denotes Ar, and in which Ar represents phenyl, phenoxyphenyl, phenylthiophenyl, 2-thienyl or 2-furyl, these radicals being optionally monosubstituted or disubstituted by fluorine, chlorine, alkyl groups having 1-6 carbon atoms, cyano or trifluoromethyl, with the exception of compounds of the formula I in which $R^1$ and $R^2$ denote hydrogen, $R^3$ denotes phenyl and Ar denotes phenyl or 4-methylphenyl, which comprises

a) cyclizing a compound of the formula II

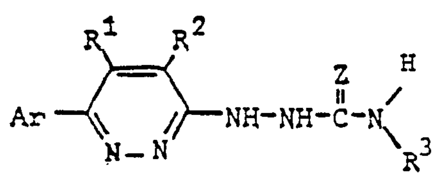

(II)

in which Ar, $R^1$, $R^2$ and $R^3$ have the meanings indicated for formula I and Z represents O or S, by heating, if appropriate with the addition of a condensation agent, to give a compound of the formula I, or

b) by reacting a compound of the formula III

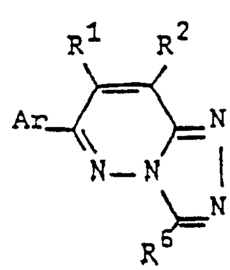

(III)

in which $R^6$ denotes chlorine, bromine or methylthio and Ar, $R^1$ and $R^2$ have the meanings indicated for formula I, with an amine of the formula IV

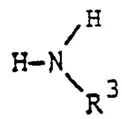

(IV)

in which $R^3$ has the meaning indicated for formula I, or

c) reacting, if appropriate with the addition of a condensation agent or catalyst, a compound of the formula V

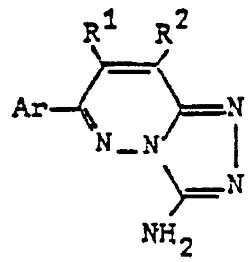

(V)

or one of its salts in which Ar, $R^1$ and $R^2$ have the meanings indicated for formula I, with a compound of the formula VI

$$R^3—Y \qquad (VI)$$

in which $R^3$ has the meaning indicated for formula I and Y represents a leaving group, such as, for example, fluorine, chlorine, bromihe, iodine,

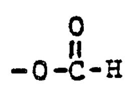

or the tosylate radical, and, if appropriate, converting a compound thus obtained into a salt thereof by adding a physiologically acceptable acid.

**Revendications** (pour les Etats contractants : BE, CH, DE, FR, GB, IT, LU, NL, SE)

1. Composés de formule I

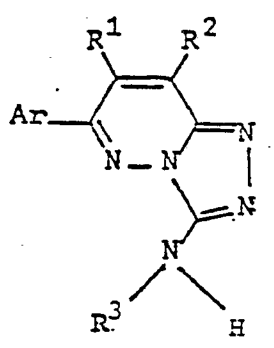

(I)

et leurs sels avec un acide physiologiquement acceptable, dans laquelle formule : $R^1$ et $R^2$ sont identiques ou différents et représentent l'hydrogène, des groupes alcoyle ayant de 1 à 6 atomes de carbone, phényle ou chloro ; $R^3$ représente un groupe alcoyle à chaîne droite ou ramifiée ayant de 1 à 6 atomes de carbone, cycloalcoyle ayant de 3 à 8 atomes de carbone, phénylalcoyle ayant de 1 à 4 atomes de carbone dans la partie alcoyle et éventuellement substitué dans la partie phényle par 1, 2 ou 3 atomes de fluor, de chlore, de brome ou d'iode ou par 1, 2 ou 3 groupes trifluorométhyle ou alcoyle ayant de 1 à 4 atomes de carbone ; un groupe alcoylcarbonyle ayant de 1 à 6 atomes de carbone dans la partie alcoyle ; un groupe cycloalcoylcarbonyle ayant de 5 à 7 atomes de carbone dans la partie cycloalcoyle ; un groupe benzoyle éventuellement substitué par 1, 2 ou 3 atomes de fluor, de chlore, de

brome ou d'iode, ou par 1, 2 ou 3 groupes trifluorométhyle ou alcoyle ayant de 1 à 4 atomes de carbone ; ou Ar ; et Ar représente un groupe phényle, phénoxyphényle, phénylthiophényle, 2-thiényle ou 2-furyle, qui peuvent être éventuellement mono- ou di-substitués par des atomes de fluor ou de chlore, ou par des groupes alcoyle ayant de 1 à 6 atomes de carbone, cyano ou trifluorométhyle, à l'exception des composés de formule I dans lesquels $R^1$ et $R^2$ représentent l'hydrogène, $R^3$ représente un groupe phényle et Ar représente un groupe phényle ou 4-méthylphényle.

2. Procédé de préparation de composés de formule I suivant la revendication 1, caractérisé en ce que :

a) on cyclise un composé de formule II

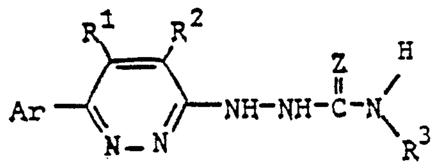

(II)

dans laquelle Ar, $R^1$, $R^2$ et $R^3$ ont les significations indiquées pour la formule I et Z est O ou S, par chauffage, en ajoutant éventuellement un agent de condensation, pour obtenir un composé de formule I, ou

b) on fait réagir un composé de formule III

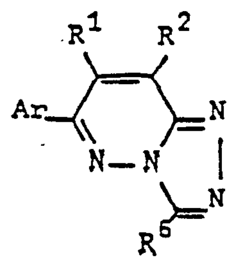

(III)

dans laquelle $R^6$ représente le chlore, le brome ou un groupe méthylthio, et Ar, $R^1$ et $R^2$ ont les significations indiquées pour la formule I, avec des amines de formule IV

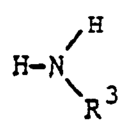

(IV)

dans laquelle $R^3$ a la signification indiquée pour la formule I, ou

c) on fait réagir un composé de formule V ou un de ses sels

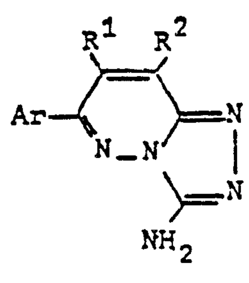

(V)

où Ar, $R^1$ et $R^2$ ont les significations indiquées pour la formule I, avec un composé de formule VI

$R^3$—Y (VI)

dans laquelle $R^3$ a la signification indiquée pour la formule I et Y représente un groupe éliminable tel que par exemple le fluor, le chlore, le brome, l'iode,

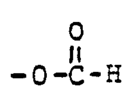

ou le groupe tosylate, éventuellement en présence d'un agent de condensation ou d'un catalyseur, et éventuellement on transforme un composé de formule I ainsi obtenu en son sel par addition d'un acide physiologiquement acceptable.

3. Composition pharmaceutique ayant une activité anxiolytique et anticonvulsive, caractérisée en ce qu'elle contient un composé de formule I suivant la revendication 1.

4. Composés de formule I suivant la revendication 1 pour le traitement des états d'excitation, de l'insomnie, des spasmes et de la dystonie végétative.

**Revendication** (pour l'Etat contractant AT)

Procédé de préparation de composés de formule I

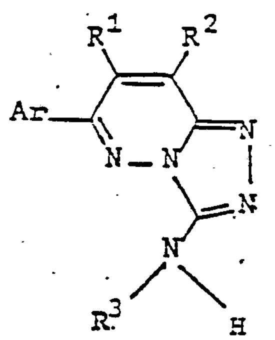

(I)

et leurs sels avec un acide physiologiquement acceptable, dans laquelle formule : $R^1$ et $R^2$ sont identiques ou différents et représentent l'hydrogène, des groupes alcoyle ayant de 1 à 6 atomes de carbone, phényle ou chloro ; $R^3$ représente un groupe alcoyle à chaîne droite ou ramifiée ayant de 1 à 6 atomes de carbone, cycloalcoyle ayant de 3 à 8 atomes de carbone, phénylalcoyle ayant de 1 à 4 atomes de carbone dans la partie alcoyle et éventuellement substitué dans la partie phényle par 1, 2 ou 3 atomes de fluor, de chlore, de brome ou d'iode ou par 1, 2 ou 3 groupes trifluorométhyle ou alcoyle ayant de 1 à 4 atomes de carbone ; un groupe alcoylcarbonyle ayant de 1 à 6 atomes de carbone dans la partie alcoyle ; un groupe cycloalcoylcarbonyle ayant de 5 à 7 atomes de carbone dans la partie cycloalcoyle ; un groupe benzoyle éventuellement substitué par 1, 2 ou 3 atomes de fluor, de chlore, de brome ou d'iode, ou par 1, 2 ou 3 groupes trifluorométhyle ou alcoyle ayant de 1 à 4 atomes de carbone ; ou Ar ; et Ar représente un groupe phényle, phénoxyphényle, phénylthiophényle, 2-thiényle ou 2-furyle, qui peuvent être éventuellement mono- ou di-substitués par des atomes de fluor, ou de chlore, ou par des groupes alcoyle ayant de 1 à 6 atomes de carbone, cyano ou trifluorométhyle à l'exception des composés de formule I dans lesquels $R^1$ et $R^2$ représentent l'hydrogène $R^3$ représente un groupe phényle et Ar représente un groupe phényle ou 4-méthylphényle, caractérisé en ce que

a) on cyclise un composé de formule II

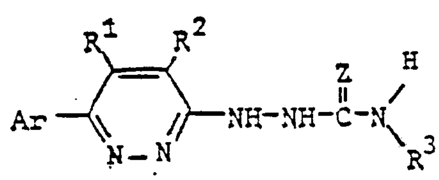

(II)

dans laquelle Ar, $R^1$, $R^2$ et $R^3$ ont les significations indiquées pour la formule I et Z est O ou S, par chauffage, en ajoutant éventuellement un agent de condensation, pour obtenir un composé de formule I, ou

b) on fait réagir un composé de formule III

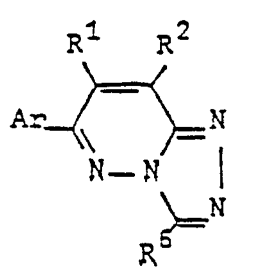

(III)

dans laquelle $R^6$ représente le chlore, le brome ou un groupe méthylthio, et Ar, $R^1$ et $R^2$ ont les significations indiquées pour la formule I, avec des amines de formule IV

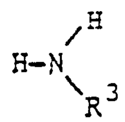

(IV)

dans laquelle $R^3$ a la signification indiquée pour la formule I, ou

c) on fait réagir un composé de formule V ou un de ses sels

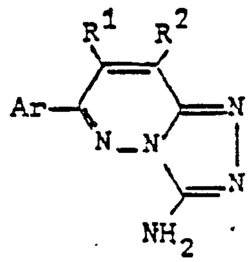

(V)

où Ar, $R^1$ et $R^2$ ont les significations indiquées pour la formule I, avec un composé de formule VI

$R^3$—Y (VI)

dans laquelle $R^3$ a la signification indiquée pour la formule I et Y représente un groupe éliminable tel que par exemple le fluor, le chlore, le brome, l'iode,

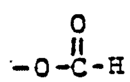

ou le groupe tosylate, éventuellement en présence d'un agent de condensation ou d'un catalyseur, et éventuellement on transforme un composé de formule I ainsi obtenu en son sel par addition d'un acide physiologiquement acceptable.